# EUROPEAN PATENT APPLICATION

(11) **EP 3 610 779 A1**
(43) Date of publication of application: **19.02.2020**
(21) Application number: 18802000.2
(22) Date of filing: 16.04.2018
(51) Int. Cl.: A61B 1/045, A61B 1/00, G02B 23/24

(54) **IMAGE ACQUISITION SYSTEM, CONTROL DEVICE, AND IMAGE ACQUISITION METHOD**

(30) Priority: 17.05.2017 JP 2017098126
(71) Applicant: Sony Corporation, Tokyo 108-0075 (JP)
(72) Inventor: TAKAHASHI, Kenji, Tokyo 108-0075 (JP); IKEDA, Kenji, Tokyo 108-0075 (JP); KASHIMA, Koji, Tokyo 108-0075 (JP); TAKEMOTO, Masaya, Tokyo 108-0075 (JP); SHIRAKI, Hisakazu, Tokyo 108-0075 (JP); MIZUKURA, Takami, Tokyo 108-0075 (JP)
(74) Representative: D Young & Co LLP
(86) International application number: PCT/JP2018/015694
(87) International publication number: WO 2018/211885

(57) **Abstract**

[Object] To provide an image acquisition system, a control apparatus, and an image acquisition method that are capable of providing an appropriate image to a practitioner and an assistant by using information regarding a medical tool.

[Solving Means] An image acquisition system includes: a light source unit; an imaging unit; a medical tool detection unit; and an image processing unit. The light source unit applies, to an affected part in which a medical tool that emits fluorescence by irradiation of excitation light is present, normal light and the excitation light. The imaging unit images the affected part under irradiation with the excitation light and under irradiation with the normal light, and acquires a first image signal obtained by imaging the fluorescence and a second image signal obtained by imaging the normal light. The medical tool detection unit detects, on the basis of the first image signal, an area in which the medical tool is present in a first image obtained from the first image signal. The image processing unit generates a display image using a result of detecting the area in which the medical tool is present and the second image signal.

## Description

### Technical Field

The present technology relates to an image acquisition system, a control apparatus, and an image acquisition method that make it possible to provide an optimal image to a practitioner when acquiring a medical image.

### Background Art

In an endoscope system, it has been proposed to make it easier to visually find the position of a medical tool by making a medical tool to be disposed in an affected part contain a luminescent agent (see, for example, Patent Literature 1). Further, it has been proposed to acquire information regarding the distance between the end of an endoscope insertion portion and an observed portion using a treatment tool provided with a mark formed of a fluorescent material that emits fluorescence by irradiation with excitation light (see, for example, Patent Literature 2).

### Citation List

### Patent Literature

Patent Literature 1: Japanese Patent Application Laid-open No. 2012-115535
Patent Literature 2: Japanese Patent Application Laid-open No. 2011-136005

### Disclosure of Invention

### Technical Problem

In general, a fluorescence image obtained by imaging under special light irradiation is wholly darker than a normal light image obtained by imaging under normal light irradiation, which makes it difficult to check an affected part in detail in some cases. Further, in the case where, for example, automatic exposure control is performed on a captured image of an affected part where a medical tool is disposed, automatic exposure control is performed on the basis of the brightness of the entire imaging area including the medical tool, and thus, the entire image becomes dark and an appropriate image is not displayed in some cases.

In view of the circumstances as described above, it is an object of the present technology to provide an image acquisition system, a control apparatus, and an image acquisition method that are capable of providing an appropriate image to a practitioner and an assistant by using information regarding a medical tool.

### Solution to Problem

In order to achieve the above-mentioned object, an image acquisition system according to the present technology includes: a light source unit; an imaging unit; a medical tool detection unit; and an image processing unit.

The light source unit applies, to an affected part in which a medical tool that emits fluorescence by irradiation with excitation light is present, normal light and the excitation light.

The imaging unit images the affected part under irradiation with the excitation light and under irradiation with the normal light, and acquires a first image signal obtained by imaging the fluorescence and a second image signal obtained by imaging the normal light.

The medical tool detection unit detects, on a basis of the first image signal, an area in which the medical tool is present in a first image obtained from the first image signal.

The image processing unit generates a display image using a result of detecting the area in which the medical tool is present and the second image signal.

With such a configuration, the area in which the medical tool is disposed is detected on the basis of the first image signal obtained by imaging under irradiation with excitation light. Then, using the result of detecting the area in which the medical tool is present, it is possible to provide an appropriate display image corresponding to the situation to a practitioner and an assistant.

The image processing unit may set, on a basis of the result of detecting the area in which the medical tool is present, an area in which the medical tool is present in a second image obtained from the second image signal, and generate the display image by performing image processing on the area in which the medical tool is present in the second image or an area other than the area in which the medical tool is present the second image.

With such a configuration, the area in which the medical tool is present in the second image obtained by imaging under irradiation with normal light is set on the basis of the result of detecting the area in which the medical tool is present. Since the area in which the medical tool is present in the second image is set, it is possible to perform image processing on the area in which the medical tool is present without being affected by information regarding the area other than the area in which the medical tool is present. Further, it is possible to perform image processing on the affected part, which is the area other than the area in which the medical tool is present in the second image, without being affected by information regarding the area in which the medical tool is present. As a result, it is possible to provide an appropriate image to a practitioner and an assistant.

The image processing unit may generate the display image by performing image processing of correcting image shake of the area in which the medical tool is present in the second image or the area other than the area in which the medical tool is present in the second image.

As a result, it is possible to correct image shake of the area in which the medical tool is present without being affected by information regarding the area other than the area in which the medical tool is present. Further, it is possible to correct image shake of the area other than the area in which the medical tool is present without being affected by information regarding the area in which the medical tool is present.

The image acquisition system may further include: a lens unit that condenses the excitation light and the normal light on the imaging unit; and a control unit that controls driving of the lens unit, in which the image processing unit calculates an automatic focus detection value in the area other than the area in which the medical tool is present in the second image, and the control unit controls driving of the lens unit on a basis of the calculated automatic focus detection value.

As a result, it is possible to perform focus control on the area other than the area in which the medical tool is present without being affected by information regarding the area in which the medical tool is present. Since the focus control is performed for imaging as described above, it is possible to obtain a display image on which image processing has been performed so that the affected part, which is the area other than the area in which the medical tool is present, is brought into focus.

The image acquisition system may further include: a lens unit that condenses the excitation light and the normal light on the imaging unit; and a control unit that controls driving of the lens unit, in which the image processing unit calculates an automatic exposure detection value in the area other than the area in which the medical tool is present in the second image, and the control unit controls driving of the lens unit on a basis of the calculated automatic exposure detection value.

As a result, it is possible to perform exposure control on the area other than the area in which the medical tool is present without being affected by information regarding the area in which the medical tool is present. Since the exposure control is performed for imaging as described above, it is possible to obtain a display image on which image processing has been performed so that an image of the affected part, which is the area other than the area in which the medical tool is present, is displayed with appropriate brightness.

The image processing unit may generate the display image by performing image processing for color correction on the area in which the medical tool is present in the second image.

As a result, it is possible to obtain a display image in which the position of the medical tool is easy to visually confirm.

The image processing unit may generate the display image by performing image processing for color correction on the area in which the medical tool is present in the second image.

As a result, it is possible to obtain a display image on which color correction has been performed on the affected part without being affected by information regarding the area in which the medical tool is present.

The image processing unit may generate the display image by performing image processing of superimposing the first image and the second image.

As a result, it is possible to obtain a display image on which both information in the first image and information in the second image have been reflected in the area in which the medical tool is disposed.

A control apparatus according to the present technology includes a medical tool detection unit; and an image processing unit.

The medical tool detection unit detects, on a basis of a first image signal obtained by imaging an affected part under irradiation with excitation light, an area in which a medical tool is present in a first image obtained from the first image signal, the medical tool being present in the affected part and emitting fluorescence by irradiation with the excitation light.

The image processing unit generates a display image using a result of detecting the area in which the medical tool is present and a second image signal obtained by imaging the affected part under irradiation with normal light.

An image acquisition method according to the present technology includes: acquiring a first image signal by imaging an affected part under irradiation with excitation light, a medical tool being present in the affected part and emitting fluorescence by irradiation with the excitation light; acquiring a second image signal by imaging the affected part under irradiation with normal light; detecting an area in which the medical tool is present in a first image obtained from the first image signal; and generating a display image using a result of detecting the area in which the medical tool is present and the second image signal.

### Advantageous Effects of Invention

As described above, in accordance with the present technology, it is possible to provide an appropriate image to a practitioner and an assistant by using information regarding an area in which a medical tool is present.

It should be noted that the effect described here is not necessarily limitative and may be any effect described in the present disclosure.

### Brief Description of Drawings

[Fig. 1] Fig. 1 is a diagram describing an overview of an endoscopic image acquisition system to which the present technology is applied.
[Fig. 2] Fig. 2 is a diagram showing a configuration example of the endoscopic image acquisition system in Fig. 1.
[Fig. 3] Fig. 3 is a partial block diagram of the endoscopic image acquisition system in Fig. 1.
[Fig. 4] Fig. 4 is a schematic diagram showing a state where light from a light source apparatus is applied to an affected part for imaging in the endoscopic image acquisition system in Fig. 1.
[Fig. 5] Fig. 5 is a diagram describing an example of a system that simultaneously acquires a fluorescence image and a normal light image.
[Fig. 6] Fig. 6 is a diagram describing an example of a system that simultaneously acquires another fluorescence image and another normal light image.
[Fig. 7] Fig. 7 is a diagram describing an example of a system that simultaneously acquires still another fluorescence image and still another normal light image.
[Fig. 8] Fig. 8 is a diagram of an example of a color filter sensor used in a system in which a fluorescence image and a normal light image are simultaneously acquired.
[Fig. 9] Fig. 9 is a partial block diagram of an endoscopic image acquisition system that performs image processing for shake correction in a first embodiment.
[Fig. 10] Fig. 10 is a diagram describing a flow of processing in a shake control unit of the endoscopic image acquisition system shown in Fig. 9.
[Fig. 11] Fig. 11 is a diagram describing a method of controlling shake when a medical tool correction mode is set on and off in the shake control unit shown in Fig. 10.
[Fig. 12] Fig. 12 is a partial block diagram of an endoscopic image acquisition system in a second embodiment.
[Fig. 13] Fig. 13 is a block diagram describing an AF/AE control unit and a drive unit in the endoscopic image acquisition system shown in Fig. 12.
[Fig. 14] Fig. 14 is a diagram describing AF/AE control processing in the endoscopic image acquisition system shown in Fig. 12.
[Fig. 15] Fig. 15 is a block diagram describing an endoscopic image acquisition system that performs image processing for color correction in a third embodiment.
[Fig. 16] Fig. 16 is a diagram describing color correction processing in the endoscopic image acquisition system shown in Fig. 15.
[Fig. 17] Fig. 17 is a block diagram describing an endoscopic image acquisition system that performs image processing for superimposition processing in a fourth embodiment.
[Fig. 18] Fig. 18 is a diagram describing the superimposition processing in the endoscopic image acquisition system shown in Fig. 17.

### Mode(s) for Carrying Out the Invention

Hereinafter, embodiments according to the present technology will be described with reference to the drawings.

### <Configuration Example of Endoscopic Image Acquisition System>

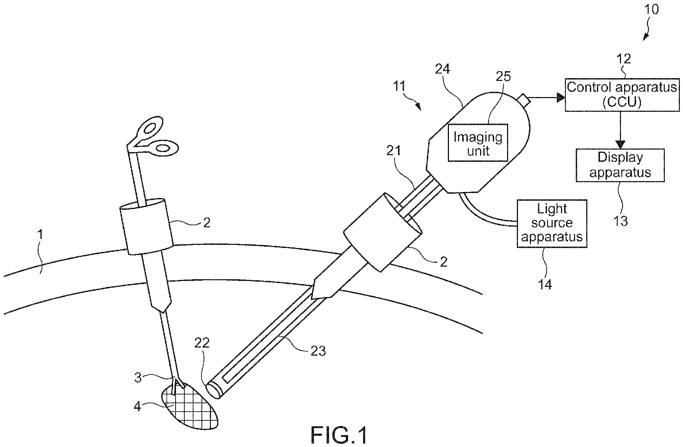

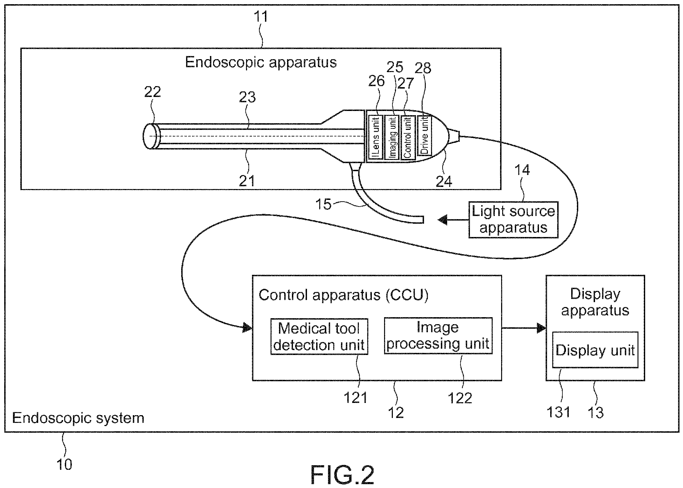

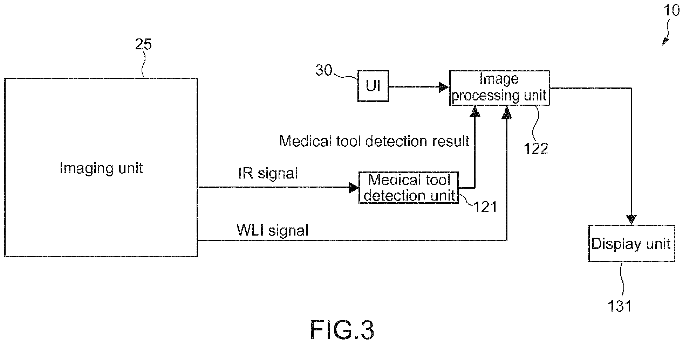

Fig. 1 is a diagram describing an overview of an endoscopic image acquisition system as an image acquisition system to which the present technology is applied. Fig. 2 is a diagram showing a configuration example of the endoscopic image acquisition system, and Fig. 3 is a partial block diagram thereof. This endoscopic image acquisition system is used in laparoscopic surgery performed in place of existing laparotomy in a medical field in recent years.

In laparoscopic surgery, in the case of performing abdominal surgery, for example, instead of cutting an abdominal wall 1 to open the stomach, which has been conventionally performed, several opening devices called trocars 2 are attached to the abdominal wall 1, and a laparoscope (hereinafter, referred to as endoscopic apparatus) 11 and a medical tool 3 are inserted into a body through holes provided in the trocars 2. Then, while viewing in real time an image of an affected part (tumor or the like) 4 video-imaged by the endoscopic apparatus 11 by a display apparatus 13, a treatment such as excision of the affected part 4 by the medical tool 3 is performed. In the endoscopic apparatus 11 having a linear rod shape, a head portion 24 is held by a practitioner, an assistant, a scopist, a robot, or the like.

An endoscopic image acquisition system 10 includes the endoscopic apparatus 11, a camera control unit (CCU) 12 as a control apparatus, the display apparatus 13, and a light source apparatus 14 as a light source unit.

The endoscopic apparatus 11 and the CCU 12 may be connected wirelessly in addition to being connected via a cable. Further, the CCU 12 may be disposed at a place away from an operating room, and may be connected via a network such as a local LAN and the Internet. The same applies to connection between the CCU 12 and the display apparatus 13.

The endoscopic apparatus 11 includes a lens barrel portion 21 having a linear rod shape, and the head portion 24. The lens barrel portion 21 is also referred to as an optical viewing tube or a rigid tube, and has a length of approximately several tens of centimeters. An objective lens 22 is provided at one end of the lens barrel portion 21, which is on the side to be inserted in to the body, and the other end of the lens barrel portion 21 is connected to the head portion 24. An optical lens unit 23 of a relay optical system is provided inside the lens barrel portion 21. Note that the shape of the lens barrel portion 21 is not limited to the linear rod shape.

The head portion 24 includes an imaging unit 25, a lens unit 26, a control unit 27, and a drive unit 28, and has a function of an imaging apparatus.

The lens unit 26 is an optical system provided at a connection portion with the lens barrel portion 21. Observation light taken from the end of the lens barrel portion 21 is guided to the head portion 24 to enter the lens unit 26. The lens unit 26 is configured by combining a plurality of lenses including a zoom lens and a focus lens. The optical characteristics of the lens unit 26 are adjusted so that the observation light is condensed on the light receiving surface of the imaging element of the imaging unit 25. Further, the zoom lens and the focus lens are each configured such that the position thereof on the optical axis is movable in order to adjust the magnification and focus of the captured image.

The imaging unit 25 is, for example, an imaging element such as a CMOS (Complementary Metal Oxide Semiconductor) image sensor, and converts the optical image of an affected part input from the lens barrel portion 21 into an image signal at a predetermined frame rate. In the CMOS image sensor, each pixel includes one photodiode and a switch using a CMOS transistor.

The imaging element detects the object luminance, i.e., performs photoelectric conversion into image signals of R, G, and B components in accordance with the amount of light of the object light image. Typically, as the imaging element, for example, a color filter sensor, which is capable of performing color imaging, in a Bayer array obtained by two-dimensionally arranging a plurality of pixels each including a photodiode in a matrix, and arranging, for example, R (red), G (green), and B (blue) color filters having different spectral characteristics on the receiving surfaces of the pixels at the ratio of 1:2:1 is used. The imaging element converts an optical image of an object into analog electrical signal (image signal) of each of R, G, and B color components, and generates an image signal of each color.

The control unit 27 controls driving of the lens unit 26.

The drive unit 28 appropriately moves, on the basis of a control signal having information for designating the magnification and focus of the captured image from the control unit 27, the zoom lens and the focus lens of the lens unit 26.

In the endoscopic apparatus 11, the optical image of the affected part 4 condensed by the objective lens 22 enters the imaging unit 25 of the head portion 24 via the optical lens unit 23, is converted into an image signal with a predetermined frame rate by the imaging unit 25, and is output to the CCU 12.

Further, the light source apparatus 14 is connected to the endoscopic apparatus 11. The endoscopic apparatus 11 emits light necessary for imaging, which is emitted from the light source apparatus 14, from the end of the lens barrel portion 21 via a light guide 15 to the affected part 4 where the medical tool 3 is present. At this time, the light source apparatus 14 is capable of switching and emitting light having various wavelengths, and is also capable of emitting special light that allows the affected part 4 to be particularly identified in addition to normal light. Therefore, the imaging unit 25 is capable of imaging an affected part under irradiation with excitation light and under irradiation with normal light to acquire a fluorescence image signal that is a first image signal obtained by imaging fluorescence and a normal light image signal that is a second image signal obtained by imaging normal light.

A fluorescent material is applied to the medical tool 3. Alternatively, the medical tool 3 may be formed of a material including a fluorescent material. Such a medical tool 3 containing a fluorescent material emits light by irradiation with excitation light. For example, ICG (indocyanine green) is used as the fluorescent agent, and light in the vicinity of 750 to 800 nm is used as the excitation light. The medical tool 3 containing the fluorescent agent emits fluorescence in the vicinity of 805 to 830 nm by irradiation with excitation light.

Examples of the medical tool that emits fluorescence by irradiation with excitation light include artifacts that do not exist in the body such as an electric knife, forceps, a catheter tube, a drain tube, yarn, and gauze. Note that although ICG is taken as an example of the fluorescent agent here, the present technology is not limited thereto, a known fluorescent agent that does not affect the human body can be used, and the medical tool 3 can be configured to emit light by irradiation with special light (excitation light).

The light source apparatus 14 is configured to be capable of emitting excitation light that is special light that cause the medical tool 3 to fluoresce, and normal light. The normal light is, for example, white light (visible light).

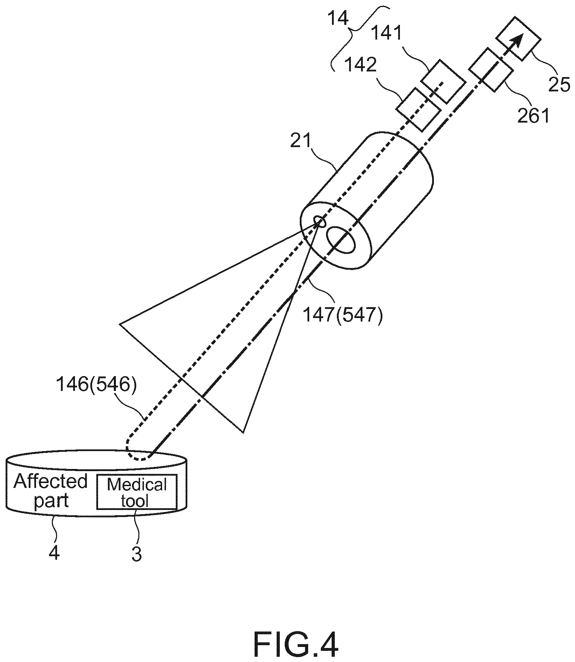

Fig. 4 is a schematic diagram showing a state where light from the light source apparatus 14 is applied to the affected part 4 where the medical tool 3 is present for imaging by the head portion 24 that is an imaging apparatus. As shown in Fig. 4, the light source apparatus 14 includes a near-infrared (NIR) bandpass filter 142 and a light source 141.

The light source 141 emits the normal light (white light). As the light source 141, a xenon lamp, a white LED (Light Emitting Diode), or the like can be used. The NIR bandpass filter 142 disposed between the light source 141 and the lens barrel portion 21 of the endoscopic apparatus 11 is a filter that causes excitation light having an excitation wavelength of the fluorescent agent to be transmitted therethrough, and is configured to be arrangeable on the optical path of the light emitted from the light source 141.

For example, in the case where the medical tool 3 contains ICG, the light source apparatus 14 is configured to emit, as excitation light, near-infrared light in the vicinity of 750 to 800 nm for exciting ICG.

In the case of applying excitation light to the affected part 4 where the medical tool 3, which is an object, is present, the NIR bandpass filter 142 is disposed on the optical path. The normal light emitted from the light source 141 passes through the NIR bandpass filter 142 to become excitation light, and enters the lens barrel portion 21. An excitation light 146 that has passed through the lens barrel portion 21 is applied to the affected part 4 where the medical tool 3 is present.

The medical tool 3 that is present in the affected part 4 emits fluorescence by the applied excitation light 146. A light 147 that includes fluorescence and reflected light reflected by the affected part 4 enters the lens barrel portion 21. The light 147 that has entered the lens barrel portion 21 passes through an excitation light cut filter 261 disposed in the lens barrel portion 21, and only a fluorescent component is extracted therefrom and enters the imaging unit 25.

In the case of applying white light to the affected part 4 where the medical tool 3 is present, the NIR bandpass filter 142 is not disposed on the optical path. A white light 546 emitted from the light source 141 passes through the lens barrel portion 21 and is applied to the affected part 4. The white light applied to the affected part 4 is reflected by the affected part 4, and a reflected light 547 enters the lens barrel portion 21. The light 547 that has entered the lens barrel portion 21 passes through the excitation light cut filter 261 disposed in the lens barrel portion 21, and enters the imaging unit 25.

Note that the light source apparatus 14 is not limited to the above-mentioned configuration. For example, although normal light and excitation light can be obtained from one light source 141 in the above description, two light sources, i.e., a light source that emits normal light and a light source that emits excitation light, may be used.

As shown in Fig. 1 to Fig. 3, the imaging unit 25 acquires, by photoelectric conversion, a fluorescence image signal (corresponding to the IR (infrared) signal in Fig. 3) as the first image signal obtained by imaging under irradiation with excitation light, and a normal light image signal (corresponding to the WLI (white light imaging) signal in Fig. 3) as the second image signal obtained by imaging under shite light (normal light). The normal light image signal and the fluorescence image signal acquired by the imaging unit 25 are output to the CCU 12.

The CCU 12 as a control apparatus includes a medical tool detection unit 121 and an image processing unit 122. The CCU 12 transmits/receives various types of information to/from the head portion 24. The fluorescence image signal (IR signal) and the normal light image signal (WLI signal) photoelectrically converted by the imaging unit 25 are input to the CCU 12.

The medical tool detection unit 121 detects, on the basis of first image signal, an area in which a medical tool is present in the fluorescence image that is the first image obtained from the first image signal. More specifically, the medical tool detection unit 121 preprocesses the fluorescence image signal, and detects, on the basis of the preprocessed fluorescence image signal, an area in which the medical tool 3 is present in the fluorescence image that is the first image obtained from the fluorescence image signal. The detection of the medical tool will be described below.

The image processing unit 122 generates a display image using the metical tool detection result (result of detecting an area in which a medical tool is present) of the medical tool detection unit 121, and the second image signal.

More specifically, the image processing unit 122 sets, on the basis of the detection result of a medical tool, an area in which a medical tool is present in the normal light image as the second image obtained from the second image signal. Then, a display image is generated by performing image processing on the area in which a medical tool is present in the normal light image or an area other than the area in which a medical tool is present in the second image.

The image data obtained by image processing is displayed on a display unit 131 of the display apparatus 13 as a display image. Examples of the image processing include shake correction processing, color correction processing, and superimposition processing. Further, the image processing unit 122 performs detection processing on the image signal for performing automatic focus control (AF) and automatic exposure control (AE) processing.

The display apparatus 13 includes the display unit 131 including liquid crystal or organic EL (Electro Luminescence). The display apparatus 13 receives the image data generated by the CCU 12, and displays a display image corresponding to the image data.

The endoscopic image acquisition system 10 is configured such that operation input by the practitioner and assistant can be performed via, for example, a user interface 30 including, various operation buttons, a touch panel, a remote controller, or the like provided on the CCU 12.

In an image acquisition method by the image acquisition system according to the present technology, a fluorescence image (first image) of an affected part is acquired, a normal light image (second image) of an affected part is acquired, an area in which a medical tool is present in the fluorescence image is detected, and a display image is generated using a result of detecting the area in which a medical tool is present and the normal light image.

More specifically, the area in which a medical tool is present in the normal light image is set using the result of detecting the area in which a medical tool is present, and a display image is generated by performing image processing on the area in which a medical tool is present in the normal light image or an area other than the area in which a medical tool is present in the normal light image.

In the endoscopic image acquisition system according to the present technology, a fluorescence image as a first image acquired under irradiation with excitation light and a normal light image as a second image acquired under irradiation with normal light are simultaneously acquired.

Then, an area in which the medical tool 3 is present in the fluorescence image is detected on the basis of the fluorescence image, and on the basis of the result of detecting the area in which the medical tool 3 is present, an area in which the medical tool 3 is present in the normal light image is set. A display image on which appropriate image processing has been performed using information regarding the area in which the medical tool 3 is present and the normal light image is displayed for the practitioner and assistant.

### <Example of System that Simultaneously Acquires Fluorescence Image and Normal Light Image>

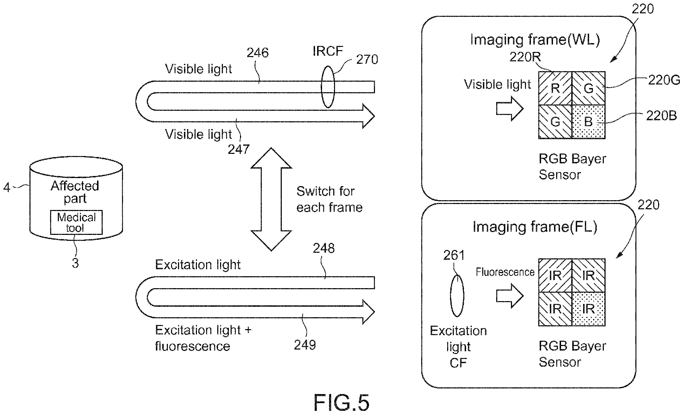

The endoscopic image acquisition system 10 is configured such that a fluorescence image and a normal light image are simultaneously acquired. Hereinafter, description will be made with reference to Fig. 5. Fig. 5 is a diagram describing an example of a system that simultaneously acquires a fluorescence image and a normal light image.

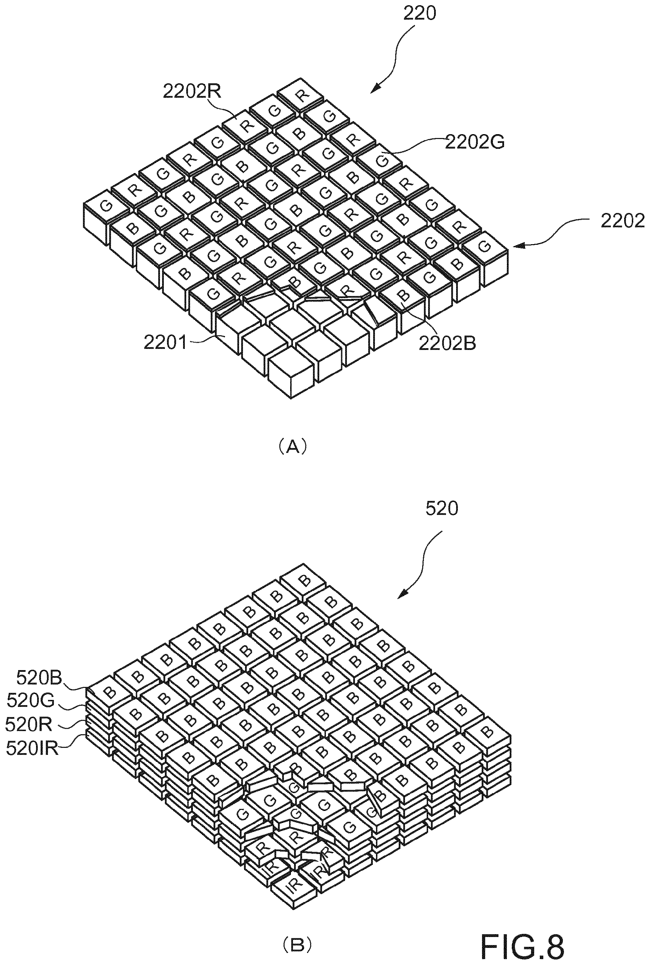

Here, as the imaging element of the imaging unit 25, a color filter sensor 220 having a Bayer array is used. Part (A) of Fig. 8 is a schematic exploded perspective view of the color filter sensor 220 having a Bayer array.

As shown in Part (A) of Fig. 8, the color filter sensor 220 is configured by forming a color selection filter 2202 above an imaging element 2201 of each pixel. The color selection filter 2202 includes a red filter 2202R, a green filter 2202G, and a blue filter 2202B.

As shown in Fig. 5, a visible light 246 that has been emitted from the light source apparatus 14 and has passed through an infrared cut filter (IRCF) 270 is applied to the affected part 4 where the medical tool 3 is present. A reflected light 247 from the affected part 4 enters the color filter sensor 220. As a result, the color filter sensor 220 acquires the normal light image signal.

As shown in Fig. 5, an excitation light 248 emitted from the light source apparatus 14 is applied to the affected part 4 where the medical tool 3 is present. A light 249 including fluorescence emitted from the medical tool 3 by the excitation light 248 and excitation light reflected by the affected part 4 passes through an excitation light cut filter (excitation light CF) 261, and an excitation light component thereof is cut to give light of a fluorescent component. The light of the fluorescent component enters the color filter sensor 220. As a result, the color filter sensor 220 acquires the fluorescence image signal.

The system shown in Fig. 5 is configured such that the visible light and the excitation light are switched for each frame and enter the color filter sensor 220, and a fluorescence image and a normal light image are captured by switching frames at high speed. By switching frames at hi speed in time division as described above, it is possible to simultaneously acquire a fluorescence image and a normal light image in a pseudo manner. Further, since the same color filter sensor 220 receives the light from the affected part 4 by irradiation with visible light and the light from the affected part 4 by irradiation with excitation light, the system configuration is simplified.

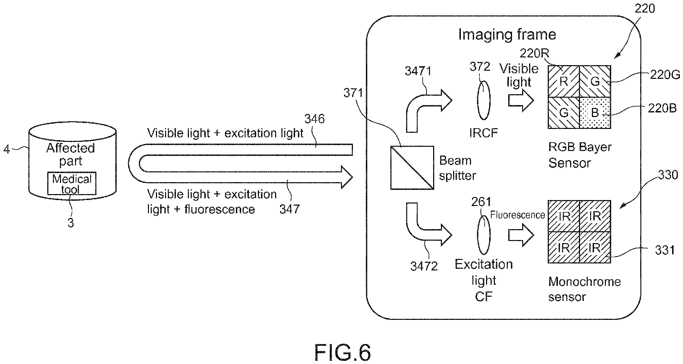

Fig. 6 is a diagram describing an example of a system that simultaneously acquires another fluorescence image and another normal light image. The same components as those in the system shown in Fig. 5 are denoted by the same reference symbols. In the system shown in Fig. 5, the light source apparatus has been configured to be capable of emitting two types of light, i.e., visible light and excitation light. However, in the system shown in Fig. 6, the light source apparatus is configured to emit mixed light of visible light and excitation light.

As shown in Fig. 6, when a light 346 in which visible light and excitation light are mixed is applied from the light source apparatus 14 to the affected part 4 where the medical tool 3 is present, the medical tool 3 emits fluorescence by irradiation with the excitation light, and thus, a light 347 in which the visible light, the excitation light, and the fluorescence are mixed is returned from the affected part 4.

This light 347 is split into a light 3471 for a normal light image and a light 3472 for a fluorescence image by a dichroic mirror 371 that is a beam splitter.

The split light 3471 for a normal light image passes through an IR cut filter (IRCF) 372, and an IR component thereof is cut. The light obtained by cutting the IR component enters the color filter sensor 220. As a result, the color filter sensor 220 acquires a normal light image signal.

The light 3472 for a fluorescence image split by the dichroic mirror 371 passes through an excitation light cut filter (excitation light CF) 261, and an excitation light component thereof is cut. The light obtained by cutting the excitation light component enters a monochrome sensor 330. As a result, the monochrome sensor 330 acquires the fluorescence image signal. The monochrome sensor 330 is a sensor that includes a plurality of imaging elements 331 capable of acquiring a fluorescent wavelength band (805 to 830 nm in this embodiment).

As described above, in the system configuration shown in Fig. 6, since the excitation light for a fluorescence image and the normal light for a normal light image can be simultaneously acquired, it is possible to take a fluorescence image and a normal light image that maintain simultaneity.

Further, although the example in which two sensors, i.e., the color filter sensor and the monochrome sensor, are used has been shown here, the number of sensors used for acquiring a normal light image may be increased to improve the resolution of each color, and the number of sensors is not limited.

Further, although the incident light has been split by the dichroic mirror here, the present technology is not limited thereto. For example, the dichroic mirror 371 is not necessarily need to be disposed, an IR cut filter and an excitation light cut filter may be disposed in front of the color filter sensor 220, and a visible light cut filter and an excitation light cut filter may be disposed in front of the monochrome sensor 330 to cause the light 347 in which visible light, excitation light, and fluorescence are mixed to enter each of the color filter sensor 220 and the monochrome sensor 330.

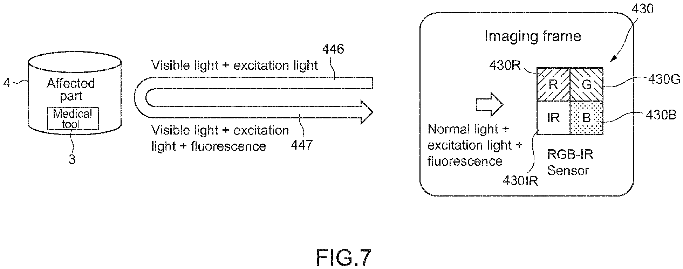

Fig. 7 is a diagram describing an example of a system that simultaneously acquires still another fluorescence image and still another normal light image. The same components as those in the system shown in Fig. 5 are denoted by the same reference symbols. In the system shown in Fig. 5, the light source apparatus has been configured to be capable of emitting two types of light, i.e., visible light and excitation light. However, in the system shown in Fig. 7, the light source apparatus is configured to emit mixed light of visible light and excitation light.

Here, as the imaging element of the imaging unit, an RGB-IR sensor 430 that is a special sensor including an IR pixel 430IR capable of acquiring a fluorescence wavelength band (805 to 830 nm in this embodiment) in addition to RGB pixels 430R, 430G, and 430B is used.

When a light 446 in which visible light and excitation light are mixed is applied from the light source apparatus to the affected part 4, a light 447 in which the visible light, the excitation light, and fluorescence are mixed is returned from the affected part 4. The light 447 enters the RGB-IR sensor 430. As a result, the RGB-IR sensor 430 simultaneously acquires both the normal light image signal and the fluorescence image signal.

As described above, in the system configuration shown in Fig. 7, since the light for a fluorescence image and the light for a normal light image can be simultaneously acquired, it is possible to take a fluorescence image and a normal light image that maintain simultaneity.

Further, although a sensor in which the RGB pixels and the IR pixel are arranged on the same surface has been described as an example in the example of the system configuration shown in Fig. 7, a stacked sensor 520 obtained by stacking four layers of RGB image sensors 520R, 520G, and 520B and an image sensor 520IR capable of acquiring a fluorescence wavelength band (805 to 830 nm in this embodiment) for each pixel as shown in Part (B) of Fig. 8 can be used. In the stacked sensor 520, R (red), G (green), B (blue), and IR (infrared light) are separated using the difference in transmission depending on the wavelength of light.

Further, instead of the color filter sensor 220 having a Bayer array, which is used in the system shown in Fig. 5 and Fig. 6, a stacked sensor obtained by stacking three layers of RGB image sensors for each pixel may be used.

Note that although description has been made on the assumption that the normal light image is a color image in the above-mentioned examples of the system that simultaneously acquires the fluorescence image and the normal light image, the present technology is not limited thereto. Depending on the application and purpose, it is displayed in a single color (gray scale), two-channel representation, or the like in some cases, and the present technology is applicable also to monochrome image display.

### <Detection of Medical Tool>

The medical tool detection unit 121 preprocesses the fluorescence image signal (IR signal) acquired by the imaging unit 25, and detects area in which a medical tool is present using the preprocessed fluorescence image signal. As the preprocessing, noise component removal processing and enhancement processing are performed.

In general, an image acquired by the imaging element has a noise component. In the case of accurately detecting an object using this image, it is necessary to remove the noise component. Further, in order to make it easy to detect an object from image information, enhancement processing for enhancing contrast and edges or enhancing color information is performed as necessary.

The fluorescence image captured under the condition of irradiation with special light tends to be darker than the normal light image captured under the condition of irradiation with normal light, and the amount of noise increases accordingly. Therefore, by performing noise component removal processing and image enhancement processing at an appropriate level before the medical tool detection unit 121 detects a medical tool, it is possible to detect the medical tool 3 more accurately.

As the noise removal processing, spatio-temporal filter processing, median filter processing, or the like can be used.

The spatio-temporal filter processing is processing of replacing the value of each pixel with the average value of points that are temporally continuous in the surrounding space. By replacing the value with the average value in the surrounding space, an image with an edge that is dull on the whole is generated.

The median filter processing is processing of replacing the value of each pixel with the median value of surrounding pixels. With this processing, it is possible to acquire an image that does not impair the edge of an input image as compared with the spatio-temporal filter.

Since the fluorescence image tends to be darker than the normal light image, it is necessary to strongly perform noise removal on the fluorescence image signal acquired during special light observation. For example, during the spatio-temporal filter processing, addition in the time direction can be increased to enhance the noise removal effect.

The medical tool detection unit 121 detects the medical tool 3 using the preprocessed fluorescence image signal. Specifically, using luminance or the like, threshold value processing is performed on the fluorescence image obtained by the preprocessing. That is, processing of extracting an area having a certain luminance or higher from the fluorescence image is performed. Then, the extracted area is determined as the area in which a medical tool is present. As the threshold value, a value set in advance may be continuously used, or may be increased or decreased by a preprocessing method. Since the environment at the time of imaging sequentially changes, the threshold value may be changed with time.

Although the medical tool 3 has been detected using the fluorescence image signal in the medical tool detection unit 121 in this embodiment, the medical tool 3 can also be detected using the normal light image signal as an auxiliary in addition to the fluorescence image signal.

### <Image Processing Using Endoscopic Image Acquisition System>

Hereinafter, imaging processing in the above-mentioned endoscopic image acquisition system 10 will be described using first to fifth embodiments as examples. In any of the embodiments, on the basis of the above-mentioned fluorescence image signal, image processing is performed using a result of detecting a medical tool (result of detecting the area in which a medical tool is present) output by the medical tool detection unit 121. Note that the configurations that have already been described in the above will be denoted the same reference symbols, and description thereof will be omitted.

### (First Embodiment)

A case where shake correction processing is performed as the image processing using the above-mentioned endoscopic image acquisition system 10 will be described.

In this embodiment, an image processing unit of an image acquisition system includes a shake control unit that generates a display image by performing image processing of correcting image shake of an area in which a medical tool is present in a second image or image shake of an area other than the area in which a medical tool is present in the second image.

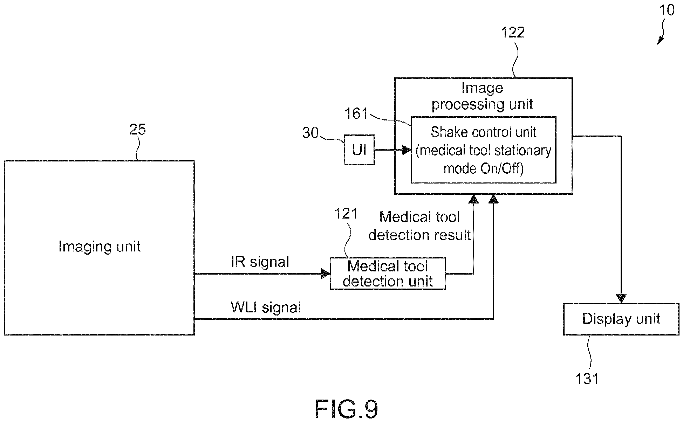

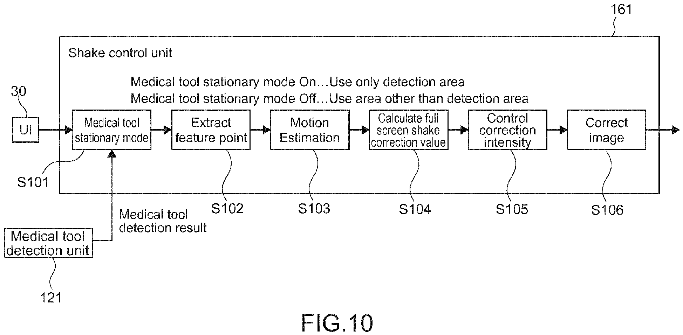

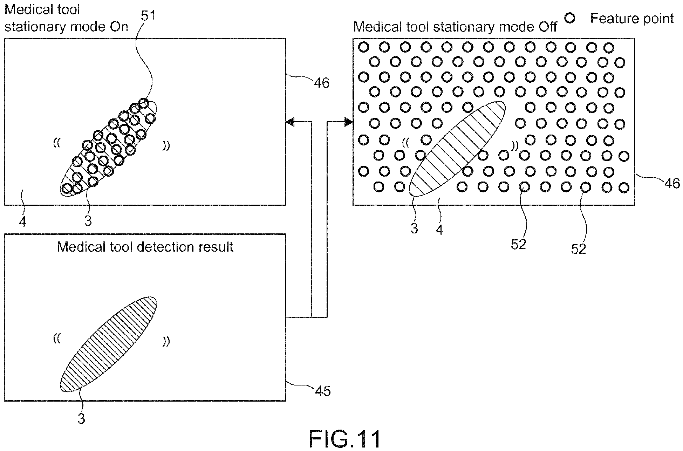

Fig. 9 is a block diagram of a main portion of the endoscopic image acquisition system according to this embodiment. Fig. 10 is a diagram describing a flow of processing in a shake control unit of the endoscopic image acquisition system according to this embodiment. Fig. 11 is a diagram describing a method of controlling shake when a medical tool correction mode is set on and off in the shake control unit.

The image processing unit 122 provided in the CCU 12 includes a shake control unit 161. In this embodiment, in the shake correction by the shake control unit 161, the result of detecting a medical tool from the medical tool detection unit 121 is used.

Since image shake can occur in an image captured by the endoscopic apparatus 11, the shake control unit 161 performs shake correction thereon. The shake control unit 161 realizes so-called camera shake correction for correcting image distortion based on image shake. In this embodiment, a practitioner and an assistant can select whether to correct image shake of the affected part 4 other than the area in which the medical tool 3 is present in the affected part 4 or to correct image shake of the medical tool 3.

In the endoscopic apparatus 11, a practitioner, an assistant, a scopist, a robot, or the like holds the head portion 24. In the case where the hand or the like holding the head portion 24 is shaken, the movement of the shake is transmitted to the objective lens 22 using the trocars 2 as a fulcrum, and thus, image shake due to the shake of the hand holding the head portion 24 can occur. This image shake is image shake of the image of the affected part 4.

Further, also the medical tool 3 is held by a practitioner, an assistant, a scopist, a robot, or the like. In the case where the hand or the like holding the medical tool 3 is shaken, the movement of the shake is transmitted to the end of the medical tool 3 using the trocars 2 as a fulcrum, and thus, image shake due to the shake of the hand holding the medical tool 3 can occur. This image shake is image shake of the image of the medical tool 3.

that is, in the case where both the head portion 24 and the medical tool 3 are vibrated, image shake in the area in which the medical tool 3 is present in the image acquired by the imaging unit 25 provided in the head portion 24 of the endoscopic apparatus 11 reflects both the shake of the head portion 24 and the shake of the medical tool 3. Meanwhile, image shake in the area other than the area in which the medical tool 3 is present in the image acquired by the imaging unit 25 provided in the head portion 24 of the endoscopic apparatus 11 reflects the shake of the head portion 24.

In this embodiment, in the case of correcting image shake of the affected part 4 in the area other than the area in which the medical tool 3 is present, the area in which the medical tool 3 is present is detected on the basis of the fluorescence image, the area in which the medical tool 3 is present in the normal light image is set using this detection result, and the image shake of the normal light image in the area other than the set area in which the medical tool 3 is present is corrected.

Meanwhile, in the case of correcting the image shake of the medical tool 3, the area in which the medical tool 3 is present is detected on the basis of fluorescence image, the area in which the medical tool 3 is present in the normal light image is set using this detection result, and the image shake of the set area in which the medical tool 3 in the normal light image is present is corrected.

As shown in Fig. 10, whether to turn on or off a medical tool stationary mode is input by the practitioner, the assistant, or the like via the user interface 30 (S101).

The medical tool stationary mode On is set in the case where the practitioner or assistant desires to correct image shake of the medical tool 3. For example, when using forceps, an electric knife, or the like as the medical tool 3, the medical tool stationary mode is turned on in the case where it is desired to check the state of the end of the forceps or electric knife on the side of the affected part 4.

The medical tool stationary mode Off is set in the case where the practitioner or assistant desires to correct image shake of the affected part 4 other than the medical tool 3. For example, the medical tool stationary mode is turned off in the case where it is desired to check the state of the organ or the like of the affected part 4 in detail.

In the case of receiving information indicating that the medical tool stationary mode is on, the shake control unit 161 performs image shake correction processing on the area in which the medical tool 3 is present in the normal light image set on the basis of the result of detecting a medical tool.

That is, as shown in Fig. 11, the medical tool detection unit 121 detects an area in which the medical tool 3 is present in a fluorescence image 45. On the basis of the detection result of the medical tool detected by the medical tool detection unit 121, the shake control unit 161 sets the area in which the medical tool 3 is present in the normal light image. Then, feature points 51 are extracted of the area in which the medical tool 3 is present in a normal light image 46 (S102).

Next, motion detection (Motion Estimation) is performed on the basis of the extracted feature points 51 (S103) As a result of the motion detection, a motion vector is obtained, and the direction of motion is determined by this motion vector. Then, a full screen shake correction value is calculated so as to correct shake of the full screen using this motion vector (S104). Next, a correction intensity control unit adjusts the correction intensity (S105), and shake correction processing is performed in accordance with the intensity (S106).

Image data obtained by performing shake correction processing as described above is output to the display apparatus 13, and the display image corresponding to the image data is displayed on the display unit 131.

Meanwhile, when receiving information indicating that the medical tool stationary mode is off, the shake control unit 161 performs image shake correction processing on the area other than the area in which the medical tool 3 is present in the normal light image 46 set on the basis of the result of detecting a medical tool.

That is, as shown in Fig. 11, the medical tool detection unit 121 detects the area in which the medical tool 3 is located in the fluorescence image 45. On the basis of the result of detecting a medical tool, the shake control unit 161 sets the area in which the medical tool 3 is present in the normal light image 46. Then, feature points 52 of the area other than the area in which the medical tool 3 is present in the normal light image 46 are extracted (S102).

After that, motion detection (Motion Estimation) is performed on the basis of the extracted feature points 52 (S103). As a result of the motion detection, a motion vector is obtained, and a full screen shake correction value is calculated on the basis of this motion vector (S104). Next, a correction intensity control unit adjusts the correction intensity (S105), and shake correction processing is performed in accordance with the intensity (S106).

Image data obtained by performing shake correction processing as described above is output to the display apparatus 13, and the display image corresponding to the image data is displayed on the display unit 131.

Here, for example, in the case where both the head portion 24 and the medical tool 3 are vibrated, in the normal light image acquired by the imaging unit 25, image shake of the medical tool 3 is shake reflecting both the shake of the head portion 24 and shake of the medical tool 3. Meanwhile, the image shake of the image area other than the medical tool 3 is shake reflecting only the shake of the head portion 24.

As described above, in the case where both the head portion 24 and the medical tool 3 are vibrated, image shake of the area in which the medical tool 3 is present and shake of the image area other than the medical tool 3 differ. In such a case, for example, if feature points are extracted in the entire area of the normal light image including the area in which the medical tool 3 is present and shake correction processing is performed, sufficient shake correction processing is not performed on both the area in which the medical tool 3 is present and the area other than the medical tool 3 in the obtained image.

Meanwhile, in this embodiment, since feature points of only the image area other than the area in which the medical tool 3 is present in the normal light image are extracted and shake correction processing is performed in the case where the medical tool stationary mode is off, it is possible to obtain an image of the affected part 4 in which image shake due to the shake of the head portion 24 is corrected without being affected by information regarding image shake of the area in which a medical tool is present. As a result, the practitioner or assistant is capable of performing an operation while viewing an appropriate display image of an affected part on which sufficient shake correction processing has been performed.

Further, in the case where the medical tool stationary mode is on, since feature points of only the area in which the medical tool 3 is present in the normal light image are extracted on the basis of the medical tool detection result of the medical tool 3 and shake correction processing is performed, it is possible to obtain a display image of the medical tool 3 in which image shake due to the shake of the head portion 24 and the shake of the medical tool 3 is corrected without being affected by information regarding image shake of the area other than the area in which a medical tool is present. As a result, the practitioner or assistant is capable of performing an operation while checking the end of the medical tool 3 in detail in the display image.

As described above, in this embodiment, the area in which the medical tool 3 is present is detected on the basis of the fluorescence image obtained by irradiating the medical tool 3 including fluorescence with excitation light, and image processing for shake correction is performed using the result of detecting the area in which a medical tool is present, thereby making it possible to provide a more appropriate image to a practitioner.

Note that although the area on which shake correction is to be performed can be selected here by the practitioner or assistant, for example, only image shake of the affected part 4 other than the area in which the medical tool 3 is present may be corrected. In such a configuration, the practitioner or assistant does not need to select an area on which shake correction is to be performed.

In such a configuration, in the image acquisition system, the image processing unit includes a shake correction unit that sets, on the basis of the result of detecting the area in which a medical tool is present, the area in which a medical tool is present in the normal light image (second image) obtained from the normal light image signal (second image signal), and performs image processing of correcting image shake of the area other than the area in which a medical tool is present in the normal light image (second image) to generate a display image.

### (Second Embodiment)

Next, a case where in the above-mentioned endoscopic image acquisition system 10, the image processing unit 122 performs detection processing on an image signal for performing automatic focus control (AF) and automatic exposure control (AE) processing using the medical tool detection result (information regarding the area in which a medical tool is present) will be described.

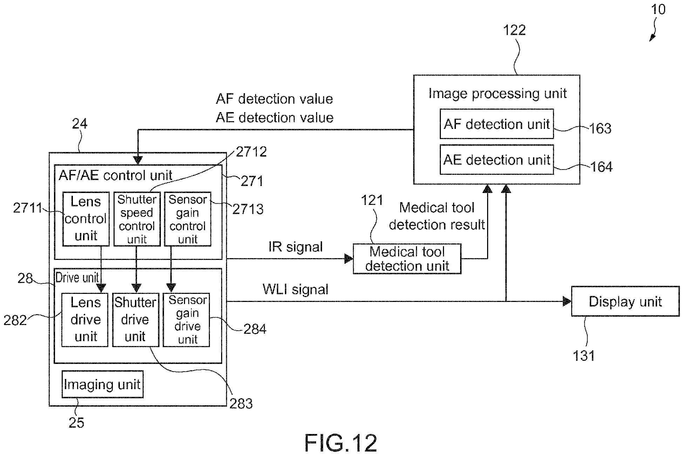

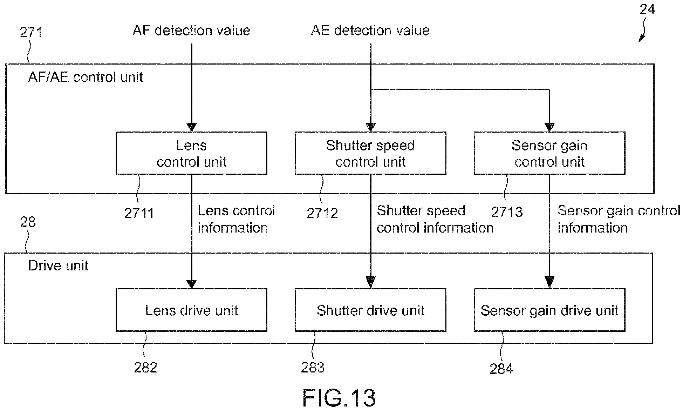

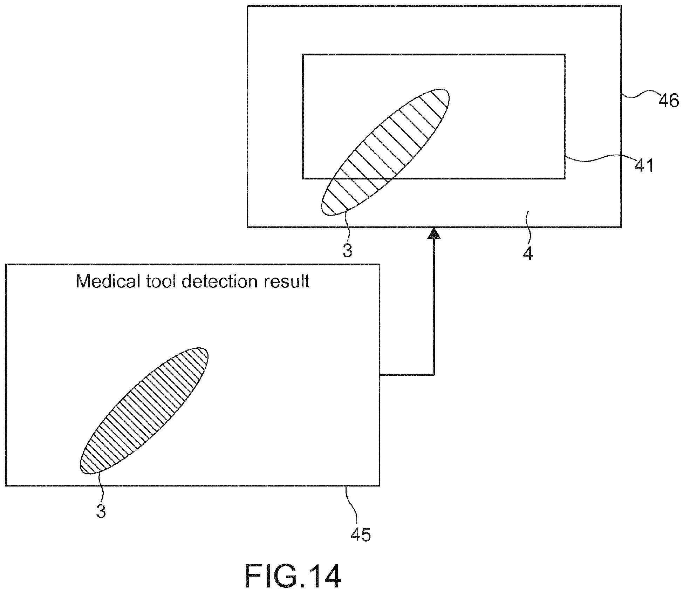

Fig. 12 is a partial block diagram of an endoscopic image acquisition system according to this embodiment. Fig. 13 is a block diagram of an AF/AE control unit and a drive unit in the endoscopic image acquisition system according to this embodiment. Fig. 14 is a diagram describing AF/AE control processing in this embodiment.

The image processing unit 122 provided in the CCU 12 includes an AF detection unit 163 and an AE detection unit 164. In this embodiment, for the detection by the AF detection unit 163 and the AE detection unit 164, the medical tool detection result from the medical tool detection unit 121 is used.

A detection frame 41 is set for the normal light image 46 by the AF detection unit 163 and the AE detection unit 164 as shown in Fig. 14, and a detection value is calculated in the detection frame 41. In this embodiment, when calculating the detection value, the detection result of the medical tool detected by the medical tool detection unit 121 is used and the area in which the medical tool 3 is present is excluded from the calculation of the detection value. Note that the detection frames having several patterns are prepared in advance.

In this embodiment, when calculating the detection value, the detection frame 41 is set for the normal light image 46 and the detection value is calculated in the area in the detection frame 41 excluding the area in which the medical tool 3 is present detected by the medical tool detection unit 121.

The detection frame 41 can be set at an arbitrary position for each AF detection and AE detection.

The AF detection unit 163 calculates the maximum value of the contrast in the detection frame 41 as the AF detection value.

The AE detection unit 164 calculates the average of the luminance in the detection frame 41 as the detection value.

As shown in Fig. 2 and Fig. 12, the head portion 24 includes the imaging unit 25, the lens unit 26, the control unit 27, and the drive unit 28. The control unit 27 includes an AF/AE control unit 271. The drive unit 28 includes a lens drive unit 282, a shutter drive unit 283, and a sensor gain drive unit 284. The AF/AE control unit 271 includes a lens control unit 2711, a shutter speed control unit 2712, and a sensor gain control unit 2713.

As shown in Fig. 12 an Fig. 13, the AF detection value and AE detection value that have been respectively calculated by the AF detection unit 163 and the AE detection unit 164 of the image processing unit 122 are output to the AF/AE control unit 271.

The AF/AE control unit 271 controls, on the basis of the input AF detection value and AE detection value, driving of a lens, a diaphragm, and a shutter that controls timing of imaging provided in the head portion 24. In the case where the AE detection value is high, the luminance of the entire captured image is reduced by increasing the shutter speed or reducing the sensor gain. In the case where the AE detection value is low, the luminance of the entire captured image is increased by decreasing the shutter speed or increasing the sensor gain.

When an object image enters the imaging element of the imaging unit 25, the imaging element captures the object image within the imaging range. That is, the imaging element performs photoelectric conversion on the optical image formed on the imaging surface and outputs an analog image signal representing the captured image.

During this imaging, the lens control unit 2711 processes the image signal (AF detection value) in the AF detection frame in the captured image to calculate a focal position such that the imaging optical system is focused on a specific object in the AF detection frame, and outputs lens control information to the lens drive unit 282.

The lens drive unit 282 drives, on the basis of an instruction from the lens control unit 2711, a focus motor and moves the focus lens of the lens unit 26, thereby causing the imaging optical system to automatically focus on a specific object.

During the above-mentioned imaging, the shutter speed control unit 2712 calculates, on the basis of the image signal (AE detection value) in the AE detection frame in the captured image, the exposure amount suitable for the image being captured, and outputs shutter speed control information to the shutter drive unit 283.

The shutter drive unit 283 supplies, on the basis of an instruction from the shutter speed control unit 2712, a timing signal to the imaging element, and the shutter speed in the imaging element is controlled at this timing.

Further, control is performed so that the exposure amount suitable for the image being captured is calculated on the basis of the AF detection value, and the opening degree of the diaphragm of the imaging optical system is adjusted. As described above, by adjusting the shutter speed and the diaphragm, exposure of the captured image is automatically controlled so that the brightness of the captured image is appropriate.

The sensor gain control unit 2713 calculates, on the basis of the AE detection value, a sensor gain, and outputs sensor gain control information to the sensor gain drive unit 254. The sensor gain drive unit 284 sets a sensor gain on the basis of an instruction from the sensor gain control unit 2713.

As described above, in this embodiment, since the area other than the area in which the medical tool 3 is present is set as the detection frame and calculation of AF detection and AE detection is performed in AF/AE control, an AF/AE error caused by the information regarding the medical tool 3 is eliminated, and it is possible to obtain an image of an affected part on which AF/AE processing suitable for observing the affected part 4 has been performed. The practitioner or assistant is capable of performing an operation while viewing an appropriate image on which AF/AE processing has been performed.

### (Third Embodiment)

Next, a case where in the above-mentioned endoscopic image acquisition system 10, color correction processing is performed as imaging processing will be described.

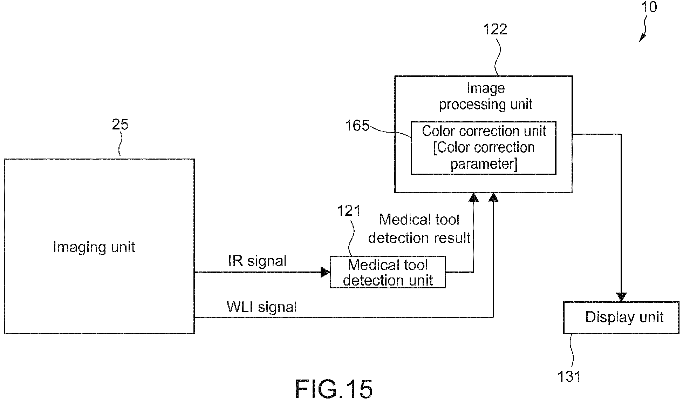

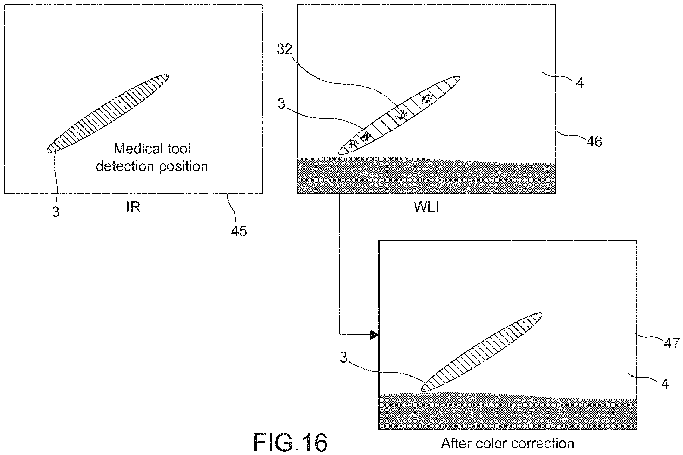

Fig. 15 is a block diagram showing a main portion of an endoscopic image acquisition system according to this embodiment. Fig. 16 is a diagram describing color correction processing in this embodiment.

In an image acquisition system according to this embodiment, an image processing unit performs image processing for color correction on the area in which a medical tool is present in a second image to generate a display image.

Further, in the image acquisition system according to this embodiment, the image processing unit performs image processing for color correction on the area other than the area in which a medical tool is present in the second image to generate a display image.

A case where image processing for color correction is performed on the area in which a medical tool is present will be described first.

As shown in Fig. 15, the image processing unit 122 provided in the CCU 12 includes a color correction unit 165. The color correction unit 165 performs color correction processing on the medical tool 3 on the basis of the color correction parameter set in advance. Examples of the color correction parameter include a parameter for converting an area in which a medical tool is present into, for example, a fixed color that is not a biological color, and a parameter for converting the color of a medical tool with reference to the color of a normal light image. In this embodiment, an example in which color correction is performed on the basis of the color correction parameter for converting into a fixed color will be described.

In this embodiment, an area in which a medical tool is present in a normal light image is set using a medical tool detection result (information regarding the area in which a medical tool is present), color correction processing is performed on the set area in which a medical tool is present on the basis of the color correction parameter to generate a display image, and the display image is displayed on the display unit 131.

As shown in Fig. 16, for example, in the case where a blood 32 is attached to the medical tool 3, only the area in which the medical tool 3 is present is detected and the blood is not visually recognized in the fluorescence image 45 obtained from the fluorescence image signal.

The color correction unit 165 sets the area in which the medical tool 3 is present in the normal light image 46 using the medical tool detection result, and performs color correction corresponding to the color correction parameter on the area in which the medical tool 3 is present in the normal light image 46. For example, in this embodiment, color correction for converting the medical tool 3 into blue, which is a fixed color, is performed. A display image 47 on which the color correction processing has been performed is displayed on the display unit 131.

Since a display image in which the area in which the medical tool 3 is present is colored with a color that is not a biological color can be obtained, the practitioner or assistant is capable of easily checking the position of the medical tool 3 in the display image. As a result, even if a medical tool such as gauze remains in the body, it is easy to find it.

Here, in the case of using, for example, general white gauze that does not contain a fluorescent material, the gauze turns red when absorbing blood, and it is difficult to distinguish it from the organ of an affected part.

Meanwhile, in this embodiment, since the medical tool contains a fluorescent material that emits fluorescence by excitation light, even if, for example, white gauze absorbs blood, an area in which a medical tool is present is detected from a fluorescence image by irradiation with excitation light, and thus, the practitioner or assistant is capable of easily checking the position of the medical tool in the affected part.

A case where a parameter for converting the color of the area in which a medical tool is present with reference to the color of the normal light image 46 is used as the color correction parameter will be described.

For example, in the case where the blood 32 is attached to the medical tool 3, the color is different between the area in which the blood 32 is attached and the area in which the blood 32 is not attached in the medical tool 3 of a normal light image. In such a case, when color correction processing is performed using the parameter for converting the color of the area in which a medical tool is present with reference to the color of the normal light image 46, the color of the medical tool 3 is converted so that a part of the medical tool 3 in which the blood 32 is attached and a part of the medical tool 3 in which the blood 32 is not attached can be distinguished from each other. At this time, a display image in which a part of the medical tool 3 in which the blood 32 is attached and a part of the medical tool 3 in which the blood 32 is not attached are colored with different colors that are not biological colors so that they can be distinguished from the affected part 4 is obtained.

As described above, in this embodiment, the area in which the medical tool 3 is present is detected on the basis of a fluorescence image obtained by irradiating the medical tool 3 containing fluorescence with excitation light, and the area in which a medical tool is present in a normal light image is set using the result of detecting a medical tool. Then, by performing color correction processing of converting the color of the area in which a medical tool is present on the normal light image, it is possible to obtain an image in which the position of a medical tool in an affected part can be easily recognized by the practitioner or assistant.

Next, a case where color correction is performed on the area other than the area in which a medical tool is present will be described.

Here, the area in which a medical tool is present in the normal light image is set using the result of detecting the area in which a medical tool is present (medical tool detection result), and a display image is generated by performing color correction so that an affected part of the area other than the set area in which a medical tool is present has a hue, lightness, and color tone that are easy for the practitioner or assistant to check, and is displayed on the display unit 131.

As a result, since color correction of an affected part is performed without being affected by information regarding a medical tool, an appropriate image is displayed.

Here, in the case of using, for example, general white gauze that does not contain a fluorescent material as a medical tool, when an affected part where the white gauze is present is imaged under irradiation with normal light, due to entering of the white object in the imaging area, color correction processing is performed in accordance with the white color to adjust the brightness. Thus, the display image becomes dark, and it is difficult for the practitioner or assistant to check the affected part in some cases.

Meanwhile, in the present technology, the area in which a medical tool is present in the normal light image can be set on the basis of the medical tool detection result, and color correction processing can be performed on the area other than the area in which a medical tool is present in the normal light image. As a result, since a medical tool is not present in the area on which color correction processing is to be performed, even if, for example, white gauze is used as a medical tool, a display image obtained by performing color correction does not become dark and the practitioner or assistant is capable of appropriately checking the affected part without losing the field of view.

### (Fourth Embodiment)

Next, a case where superimposition processing is performed in the above-mentioned endoscopic image acquisition system 10 as image processing will be described.

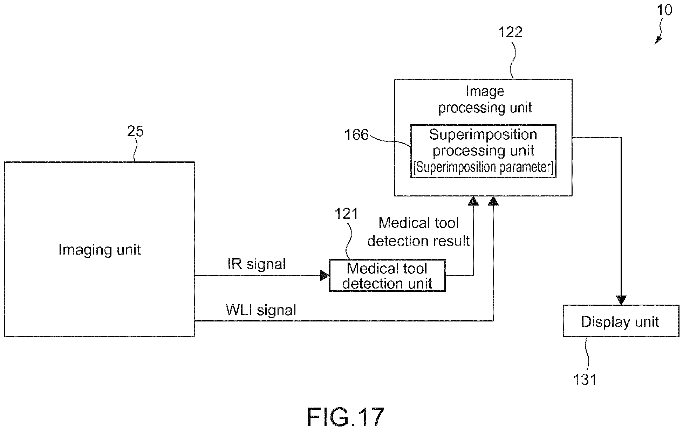

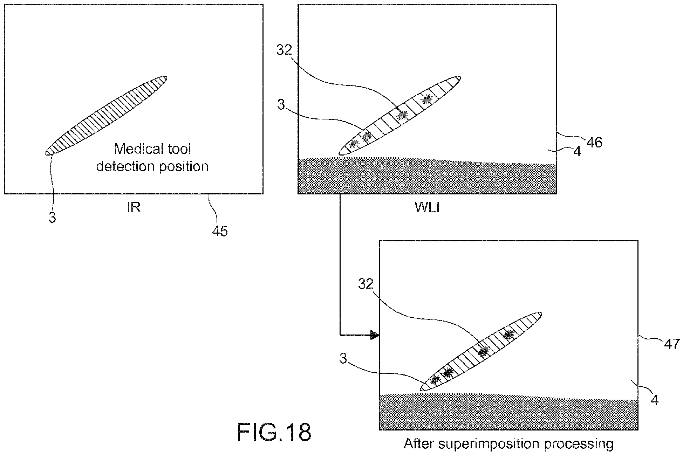

Fig. 17 is a block diagram showing a main portion of an endoscopic image acquisition system according to this embodiment. Fig. 18 is a diagram describing the superimposition processing in this embodiment.

In the image acquisition system according to this embodiment, the image processing unit includes a superimposition processing unit that generates a display image by performing image processing of superimposing the first image obtained from the first image signal and the second image obtained from the second image.

As shown in Fig. 17, the image processing unit 122 provided in the CCU 12 includes a superimposition processing unit 166. The superimposition processing unit 166 performs, on the basis of a superimposition parameter, superimposition processing of performing the fluorescence image and the normal light image. The superimposition parameter is a parameter indicating the superimposition ratio between the fluorescence image and the normal light image. The superimposition parameter is set so that the sum of a coefficient a indicating the ratio of adjustment of the pixel value of the fluorescence image and a coefficient b indicating the ratio of adjustment of the normal light image is one. For example, each of the coefficient a and the coefficient b can be 0.5.

In this embodiment, a display image is generated by performing, on the basis of the superimposition parameter, superimposition processing on the fluorescence image in which the area in which a medical tool is present is visible and the normal light image, and is displayed on the display unit 131. In other words, the area in which a medical tool is present in the normal light image that is the second image is set on the basis of the medical tool detection result, and image processing is performed on the normal light image so that the fluorescence image and the normal light image are superimposed in the area in which a medical tool is present in the normal light image.

As shown in Fig. 18, only an image of the medical tool 3 is acquired in the fluorescence image 45. In the normal light image 46 obtained from the normal light image signal, an image of the affected part 4 including the medical tool 3 is acquired.

The superimposition processing unit 166 superimposes, on the basis of the superimposition parameter, the fluorescence image 45 in which the area in which the medical tool 3 is present is imaged and the normal light image 46 to generate the display image 47. At this time, in the case where, for example, the blood 32 is attached to the medical tool 3 of the normal light image 46, the display image 47 in which the medical tool 3 to which the blood 32 is attached is present in the affected part 4 is obtained.

By superimposing the fluorescence image and the normal light image as described above, a display image in which the area in which a medical tool is present in the affected part is easy for the practitioner or assistant to recognize is obtained, and further, a display image in which display of the area in which a medical tool is present takes into account information obtained from the normal light image regarding, for example, attachment of blood, is obtained.

It should be noted that the present technology may take the following configurations.
(1) An image acquisition system, including:
   a light source unit that applies, to an affected part in which a medical tool that emits fluorescence by irradiation with excitation light is present, normal light and the excitation light;
   an imaging unit that images the affected part under irradiation with the excitation light and under irradiation with the normal light, and acquires a first image signal obtained by imaging the fluorescence and a second image signal obtained by imaging the normal light;
   a medical tool detection unit that detects, on a basis of the first image signal, an area in which the medical tool is present in a first image obtained from the first image signal; and
   an image processing unit that generates a display image using a result of detecting the area in which the medical tool is present and the second image signal.
(2) The image acquisition system according to (1) above, in which
   the image processing unit sets, on a basis of the result of detecting the area in which the medical tool is present, an area in which the medical tool is present in a second image obtained from the second image signal, and generates the display image by performing image processing on the area in which the medical tool is present in the second image or an area other than the area in which the medical tool is present the second image.
(3) The image acquisition system according to (2) above, in which
   the image processing unit generates the display image by performing image processing of correcting image shake of the area in which the medical tool is present in the second image or the area other than the area in which the medical tool is present in the second image.
(4) The image acquisition system according to (2) or (3) above, further including:
   a lens unit that condenses the excitation light and the normal light on the imaging unit; and
   a control unit that controls driving of the lens unit, in which
   the image processing unit calculates an automatic focus detection value in the area other than the area in which the medical tool is present in the second image, and
   the control unit controls driving of the lens unit on a basis of the calculated automatic focus detection value.
(5) The image acquisition system according to any one of (2) to (4) above, further including:
   a lens unit that condenses the excitation light and the normal light on the imaging unit; and
   a control unit that controls driving of the lens unit, in which
   the image processing unit calculates an automatic exposure detection value in the area other than the area in which the medical tool is present in the second image, and
   the control unit controls driving of the lens unit on a basis of the calculated automatic exposure detection value.
(6) The image acquisition system according to any one of (2) to (5) above, in which
   the image processing unit generates the display image by performing image processing for color correction on the area in which the medical tool is present in the second image.
(7) The image acquisition system according to any one of (2) to (6) above, in which
   the image processing unit generates the display image by performing image processing for color correction on the area in which the medical tool is present in the second image.
(8) The image acquisition system according to (2) above, in which
   the image processing unit generates the display image by performing image processing of superimposing the first image and the second image.
(9) A control apparatus, including:
   a medical tool detection unit that detects, on a basis of a first image signal obtained by imaging an affected part under irradiation with excitation light, an area in which a medical tool is present in a first image obtained from the first image signal, the medical tool being present in the affected part and emitting fluorescence by irradiation with the excitation light; and
   an image processing unit that generates a display image using a result of detecting the area in which the medical tool is present and a second image signal obtained by imaging the affected part under irradiation with normal light.
(10) An image acquisition method, including:
   acquiring a first image signal by imaging an affected part under irradiation with excitation light, a medical tool being present in the affected part and emitting fluorescence by irradiation with the excitation light;
   acquiring a second image signal by imaging the affected part under irradiation with normal light;
   detecting an area in which the medical tool is present in a first image obtained from the first image signal; and
   generating a display image using a result of detecting the area in which the medical tool is present and the second image signal.

### Reference Signs List

- 3: medical tool
- 4: affected part
- 10: endoscopic image acquisition system (image acquisition system)
- 12: CCU (control apparatus)
- 14: light source apparatus (optical unit)
- 25: imaging unit
- 26: lens unit
- 27: control unit
- 45: fluorescence image (first image)
- 46: normal light image (second image)
- 121: medical tool detection unit
- 122: image processing unit

## Claims

1. An image acquisition system, comprising:
a light source unit that applies, to an affected part in which a medical tool that emits fluorescence by irradiation with excitation light is present, normal light and the excitation light;
an imaging unit that images the affected part under irradiation with the excitation light and under irradiation with the normal light, and acquires a first image signal obtained by imaging the fluorescence and a second image signal obtained by imaging the normal light;
a medical tool detection unit that detects, on a basis of the first image signal, an area in which the medical tool is present in a first image obtained from the first image signal; and
an image processing unit that generates a display image using a result of detecting the area in which the medical tool is present and the second image signal.

2. The image acquisition system according to claim 1, wherein
the image processing unit sets, on a basis of the result of detecting the area in which the medical tool is present, an area in which the medical tool is present in a second image obtained from the second image signal, and generates the display image by performing image processing on the area in which the medical tool is present in the second image or an area other than the area in which the medical tool is present the second image.

3. The image acquisition system according to claim 2, wherein
the image processing unit generates the display image by performing image processing of correcting image shake of the area in which the medical tool is present in the second image or the area other than the area in which the medical tool is present in the second image.

4. The image acquisition system according to claim 2, further comprising:
a lens unit that condenses the excitation light and the normal light on the imaging unit; and
a control unit that controls driving of the lens unit, wherein
the image processing unit calculates an automatic focus detection value in the area other than the area in which the medical tool is present in the second image, and
the control unit controls driving of the lens unit on a basis of the calculated automatic focus detection value.

5. The image acquisition system according to claim 2, further comprising:
a lens unit that condenses the excitation light and the normal light on the imaging unit; and
a control unit that controls driving of the lens unit, wherein
the image processing unit calculates an automatic exposure detection value in the area other than the area in which the medical tool is present in the second image, and
the control unit controls driving of the lens unit on a basis of the calculated automatic exposure detection value.

6. The image acquisition system according to claim 2, wherein
the image processing unit generates the display image by performing image processing for color correction on the area in which the medical tool is present in the second image.

7. The image acquisition system according to claim 2, wherein
the image processing unit generates the display image by performing image processing for color correction on the area in which the medical tool is present in the second image.

8. The image acquisition system according to claim 2, wherein
the image processing unit generates the display image by performing image processing of superimposing the first image and the second image.

9. A control apparatus, comprising:
a medical tool detection unit that detects, on a basis of a first image signal obtained by imaging an affected part under irradiation with excitation light, an area in which a medical tool is present in a first image obtained from the first image signal, the medical tool being present in the affected part and emitting fluorescence by irradiation with the excitation light; and
an image processing unit that generates a display image using a result of detecting the area in which the medical tool is present and a second image signal obtained by imaging the affected part under irradiation with normal light.

10. An image acquisition method, comprising:
acquiring a first image signal by imaging an affected part under irradiation with excitation light, a medical tool being present in the affected part and emitting fluorescence by irradiation with the excitation light;
acquiring a second image signal by imaging the affected part under irradiation with normal light;
detecting an area in which the medical tool is present in a first image obtained from the first image signal; and
generating a display image using a result of detecting the area in which the medical tool is present and the second image signal.
